# EUROPÄISCHE PATENTSCHRIFT

(11) **EP 2 888 221 B1**
(45) Veröffentlichungstag und Bekanntmachung des Hinweises auf die Patenterteilung: **02.11.2016**
(21) Anmeldenummer: 13753136.4
(22) Anmeldetag: 15.08.2013
(51) Int. Cl.: C07C 51/15, C07C 57/22

(54) **VERFAHREN ZUR HERSTELLUNG VON ACETYLENDICARBONSÄURE AUS ACETYLEN UND KOHLENSTOFFDIOXID**
METHOD FOR PRODUCING ACETYLENEDICARBOXYLIC ACID FROM ACETYLENE AND CARBON DIOXIDE
PROCÉDÉ DE FABRICATION D'ACIDE DICARBOXYLIQUE ACÉTYLÉNIQUE À PARTIR D'ACÉTYLÈNE ET DE DIOXYDE DE CARBONE

(30) Priorität: 24.08.2012 EP 12181734
(43) Veröffentlichungstag der Anmeldung: 01.07.2015
(73) Patentinhaber: BASF SE, 67056 Ludwigshafen am Rhein (DE)
(72) Erfinder: ARNDT, Matthias, 67655 Kaiserslautern (DE); GOOSSEN, Lukas J, 67663 Kaiserslautern (DE)
(86) Internationale Anmeldenummer: PCT/EP2013/067090
(87) Internationale Veröffentlichungsnummer: WO 2014/029689

(56) Entgegenhaltungen:
- WO-A1-2011/075087
- WO-A1-2012/022801

## Beschreibung

Die Erfindung betrifft ein Verfahren zur Herstellung von Acetylendicarbonsäure durch Umsetzung von Acetylen mit Kohlenstoffdioxid.

Acetylendicarbonsäure wird nach einem historischen Verfahren, das auf das Jahr 1877 zurück geht, durch alkalische Eliminierung von meso-Dibrombernsteinsäure gebildet.

E. C. Horning, J. Am Chem. Soc. 1945, 67, 1412-1422 beschreiben die Bildung von Acetylendicarbonsäure mit einer Ausbeute von 34 % durch Umsetzung von Dilithiumcarbid, welches durch Reaktion von Vinylbromid mit Butyllithium erhalten wird, mit Kohlendioxid in Ether, wobei Kohlendioxid als Trockeneis eingesetzt wird.

Bei der elektrochemischen anodischen Oxidation von Alkinolen an Bleioxidelektroden kann die Acetylendicarbonsäure in einer Ausbeute von ca. 50 % erhalten werden. Ein elektrochemisches Verfahren zur Herstellung von Acetylendicarbonsäure beschreibt V. Wolf, Chem. Ber. 1954, 87, 668-676. Als Nebenreaktion läuft dabei die Decarboxylierung der Acetylendicarbonsäure unter Bildung von Kohlendioxid und Ethin ab. J. Kaulen und H.J. Schäfer, Tetrahedron Lett. 1982, 38, 3299-3308 beschreiben die Oxidation von alpha-omega-Diolen zu Dicarbonsäuren an Nickeloxid-Anoden. Die elektrochemischen Verfahren sind jedoch mit einem hohen Energieaufwand und hohen Kosten verbunden.

In WO 2012/022801 wird die direkte Carboxylierung von Acetylen in Gegenwart von (4,7-Diphenylphenanthrolin)bis(triphenylphosphin)kupfer(I) und Cäsiumcarbonat mit 5 bar CO₂ in Dimethylformamid beschrieben. Dabei wird ein Gemisch aus Acetylenmonocarbonsäure und Acetylendicarbonsäure erhalten, die in Form ihrer n-Hexylester im Reaktionsgemisch nachgewiesen werden. Der Umsatz nach zwei Stunden Reaktionsdauer bei 60 °C entspricht einer Umsatzzahl (turn over number, TON) von ca. 1,8 für die Bildung der Acetylendicarbonsäure. Die WO 2012/022801 offenbart weiterhin die Carboxylierung von terminalen Alkinen zu den entsprechenden Propiolsäuren in Gegenwart von Phenanthrolin-Phosphin-Kupfer(1)-Komplexen und Cäsiumcarbonat. WO 2011/075087 offenbart die Carboxylierung von terminalen Alkinen mit CO₂ in Gegenwart einer Kupferverbindung und einer Aminbase.

Die direkte Carboxylierung von Acetylen zu Acetylendicarbonsäuren erweist sich als schwierig, da Acetylen eine nur geringe Löslichkeit in organischen Lösungsmitteln aufweist und Kohlendioxid als Co-Reaktant aufgrund seiner deutlich höheren Löslichkeit in organischen Lösungsmitteln eine Anreicherung von Acetylen im Reaktionsmedium zusätzlich erschwert. Auch weisen die zunächst gebildeten Salze der Acetylenmonocarbonsäure eine nur geringe Löslichkeit auf, was den zweiten Carboxylierungsschritt zur Acetylendicarbonsäure erschwert, so dass Produktgemische der Acetylenmonocarbonsäure und der Acetylendicarbonsäure gebildet werden. Weiterhin tendieren die Acetylencarbonsäuren, insbesondere die Acetylendicarbonsäure, dazu, in Lösung zu decarboxylieren, wobei die Decarboxylierung zudem durch Silber-Komplexe katalysiert wird. Ferner neigt Acetylen zu Polymerisierungs- oder Vinylierungsreaktionen, wodurch die Ausbeute zusätzlich verringert werden kann.

Bei der Carboxylierung von Acetylen in Gegenwart von Silberverbindun-gen besteht zudem die Gefahr, dass sich explosive Kupfer- oder Silberacetylide bilden. Daher ist es erstrebenswert, die Menge an eingesetztem Katalysator zu minimieren. Diese Gefahr besteht immer, wenn mit Acetylen unter Druck gearbeitet wird. Je weniger Katalysator eingesetzt wird, desto geringer ist diese Gefahr.

Außerdem ist die Mitverwendung stöchiometrischer Mengen von anorganischen Basen in dem in WO 2012/022801 A1 beschriebenen Verfahren aufgrund der anfallenden Salzfracht in einem im industriellen Maßstab durchgeführten Verfahren ungünstig. Um die gebildete Acetylendicarbonsäure in einem Folgeschritt zu der wertvollen Zielverbindung Butan-1,4-diol hydrieren zu können, muss die Acetylendicarbonsäure von Cäsiumcarbonat und den anderen nach der Reaktion vorliegenden Cäsiumsalzen abgetrennt werden.

Aufgabe der Erfindung ist es, ein einfach durchzuführendes Verfahren zur Herstellung von Acetylendicarbonsäure durch direkte Carboxylierung von Acetylen bereitzustellen. Aufgabe der Erfindung ist es insbesondere, ein solches Verfahren bereitzustellen, das durch einen hohen Umsatz, bezogen auf die Menge an eingesetztem Katalysator, gekennzeichnet ist. Aufgabe der Erfindung ist es weiterhin, ein solches Verfahren bereitzustellen, das ohne die Mitverwendung von anorganischen Basen auskommt.

Gelöst wird die Aufgabe durch ein Verfahren zur Herstellung von Acetylendicarbonsäure durch Umsetzung von Acetylen mit Kohlenstoffdioxid, dadurch gekennzeichnet, dass die Umsetzung in Gegenwart eines Silbersalzes und einer Aminbase durchgeführt wird.

Überraschender Weise wurde gefunden, dass die direkte Carboxylierung von Acetylen zu Acetylendicarbonsäure in Gegenwart eines Silbersalzes auch in Abwesenheit einer anorganischen Base wie Cäsiumcarbonat verläuft, wenn in Gegenwart einer Aminbase gearbeitet wird. Dies ist umso überraschender, da Aminbasen sehr stark an Silber koordinieren und Koordinationsstellen für Acetylen und CO₂ blockieren können. Da die Löslichkeit von Kohlenstoffdioxid und insbesondere von Acetylen unter den Reaktionsbedingungen sehr gering ist, war es zu erwarten, dass der Silberkatalysator durch den großen Überschuss einer Aminbase im Vergleich zur geringen Menge an gelöstem Acetylen desaktiviert wird. Des Weiteren werden Amine in Gegenwart von CO₂ unter Bildung von Carbamaten carboxyliert, wodurch ihre Basizität verringert wird. Es ist ferner überraschend, dass die gebildete Acetylendicarbonsäure unter den Reaktionsdedingungen (geringer Kohlenstoffdioxid-Partialdruck und hohe Temperatur) stabil ist und nicht decarboxyliert.

Die direkte Carboxylierung von Acetylen zu Acetylendicarbonsäure erfolgt in Gegenwart von Silberkatalysatoren und einer Aminbase. Geeignete Silberkatalysatoren sind ausgewählt aus der Gruppe bestehend aus elementarem Silber, kolloidalen Silberpartikeln, die wahlweise stabilisierende Zusätzen wie Phosphinliganden, Dimethylsulfoxid und/oder Polyvinylpyrrolidinon enthalten können, Silber(I)halogeniden wie AgF, AgCl, AgBr und Agl, Silbernitrat, Silbertetrafluoroborat, Silbertrifluoromethansulfonat, Silbercarboxylate (Silberacetat), Silberhexafluorophosphat, Silberoxid, Silbersulfat, Silberhexafluoroantimonat, Silber-p-toluolsulfonat und Silbercarbonat. Bevorzugt sind Silber(I)iodid Agl, Silber(I)nitrat AgNO₃ und Silbertetrafluoroborat AgBF₄, besonders bevorzugt ist Silber(I)nitrat.

In einer Ausführungsform der Erfindung wird die Umsetzung von Acetylen mit Kohlenstoffdioxid in Gegenwart eines Silbersalzes, insbesondere AgNO₃, durchgeführt.

Geeignete Aminbasen sind bei der Reaktionstemperatur flüssige Aminbasen,wie Alkylamine, insbesondere Tri-C₃-C₆-alkylamine wie Tripropylamin und Tributylamin, Alkanolamine, insbesondere Mono-, Di- und Tri-C₂-C₄-alkanolamine wie Mono-, Di- und Triethanolamin, und insbesondere heterocyclische Aminbasen wie N-Methylpiperidin, N-Methylpiperidon, N-Methylmorpholin, N-Methyl-2-pyrrolidon, vor allem aber Diazabicyclononen (DBN) und Diazabicycloundecen (DBU).

Besonders bevorzugte Aminbasen sind 1,5-Diazabicyclo[4.3.0]-non-5-en (DBN) und 1,8-Diazabicyclo[5.4.0]-undec-7-en (DBU).

Die Umsetzung kann in Gegenwart eines Lösungsmittels durchgeführt werden. Geeignete Lösungsmittel sind beispielsweise Dimethylsulfoxid (DMSO), Dimethylformamid (DMF), Wasser, NMP, Dioxan, Sulfolan und Alkohole. Silberkolloide können durch Erhitzen in DMSO präformiert werden und dann dem Reaktionsgemisch zugesetzt werden. DMSO ist ein sehr gutes Lösungsmittel, außerdem bildet Silber beim Erhitzen darin sehr feine Kolloide.

Die Umsetzung kann auch in Abwesenheit eines separaten Lösungsmittels durchgeführt werden, wobei das Silbersalz in der Aminbase gelöst vorliegen kann.

Die Umsetzung wird im Allgemeinen bei einem Gesamtdruck (Acetylen und Kohlenstoffdioxid) von 1 bis 50 bar, bevorzugt 1 bis 20 bar, und einer Temperatur von im Allgemeinen 50 bis 120 °C, bevorzugt 50 bis 100 °C durchgeführt. Das molare Verhältnis von Kohlenstoffdioxid zu Acetylen beträgt dabei im Allgemeinen von 2 : 1 bis 50 :1, bevorzugt von 5 : 1 bis 20 : 1. Mit dem erfindungsgemäßen Verfahren wird eine hohe Umsatzzahl (turn over number, TON) erzielt.

In einer Ausführungsform der Erfindung wird die Umsetzung bei Normaldruck (1 bar) durchgeführt. In einer weiteren Ausführungsform wird bei einem Gesamtdruck von 1 bis 10 bar gearbeitet.

Die gebildete Acetylendicarbonsäure kann anschließend zu Butan-1,4-diol hydriert werden. Die Hydrierung kann ohne Aufarbeitung des Reaktionsgemischs der Acetylen-Carboxylierung in Gegenwart der Aminbase durchgeführt werden.

Die Erfindung wird durch die nachstehenden Beispiele näher erläutert.

### Beispiele

### Beispiele 1 bis 4

Die Versuche wurden in 100 mL Headspace-Vial für die Gaschromatographie durchgeführt, die mit Aluminium-Bördelkappen mit Teflon-beschichteten Butylgummi-Septen verschlossen wurden.

Um die Gefäße zu temperieren, wurde ein 8 cm hoher zylindrischer Aluminiumblock verwendet, dessen Durchmesser demjenigen der Heizplatten eines Labor-Magnetrührers entsprach. Der Aluminiumblock war mit 7 cm tiefen Bohrungen vom Durchmesser der Reaktionsgefäße und einer Bohrung zur Aufnahme eines Temperaturfühlers versehen.

Es wurde ein Vakuumverteiler zum Anschluss an eine Schlenk-Linie angefertigt, um ein gleichzeitiges Evakuieren und Befüllen von mehreren Gefäßen zu ermöglichen. Dazu wurden vakuumfeste Teflonschläuche mit einem Durchmesser von 3 mm an einem Ende mit Adaptern zur Aufnahme von Luer-Lock-Spritzennadeln verbunden und mit dem anderen Ende an ein Stahlrohr angeschlossen, das über einen Vakuumschlauch mit der Schlenk-Linie verbunden war.

Die festen Edukte wurden an der Luft in die Reaktionsgefäße eingewogen. Es wurden 20 mm Magnet-Rührkerne zugegeben und die Gefäße mittels einer Bördelzange mit Septumkappen luftdicht verschlossen. Anschließend wurden die Reaktionsgefäße in die Bohrungen des Aluminiumblocks gesteckt und über Hohlnadeln durch die Septumkappen mit dem Vakuumverteiler verbunden.

Um eine Inergasatmosphäre in den Reaktionsgefäßen herzustellen, wurden diese dreimal hintereinander evakuiert und zweimal mit Stickstoff und anschließend mit Kohlendioxid rückbefüllt. Flüssige Reagenzien wurden mit Spritzen durch die Septumkappen zugegeben.

Die Beispiele 1 und 2 wurden bei 5 bar Druck durchgeführt. Dazu wurden die Nadeln des Vakuumverteilers unter CO₂-Gegenstrom entfernt und die Reaktionsgefäße in einen Autoklaven-Reaktor überführt. Anschließend wurden lange, verdrillte Kanülen in die Septen der Reaktionsgefäße gestochen und der Autoklaven-Reaktor verschlossen. Über ein Gasventil wurde die Atmosphäre durch drei Vakuum-CO₂-Zyklen ausgetauscht. Anschließend wurden 1 bar Acetylen und 4 bar CO₂ auf den Autoklaven-Reaktor aufgepresst. Es wurde 16 h bei 60 °C mit ca. 700 Umdrehungen pro Minute gerührt.

Die Beispiele 3 und 4 (Edukt 1-Oktin) wurden bei 20 bar Druck durchgeführt. Dazu wurden die Nadeln des Vakuumverteilers unter CO₂-Gegenstrom entfernt und die Reaktionsgefäße in einen Autoklaven-Reaktor überführt. Anschließend wurden lange, verdrillte Kanülen in die Septen der Reaktionsgefäße gestochen und der Autoklaven-Reaktor verschlossen. Über ein Gasventil wurde die Atmosphäre durch drei Vakuum-CO₂-Zyklen ausgetauscht. Anschließend wurden 20 bar CO₂ auf den Autoklaven-Reaktor aufgepresst. Es wurde 16 h bei 40 °C mit ca. 700 Umdrehungen pro Minute gerührt.

Nach Ablauf der Reaktionszeit und Abkühlen der Gefäße wurde entweder (1) eine Substanz zur Veresterung (1-Bromhexan oder Methyliodid) zugegeben und n-Tetradecan als interner Standard injiziert, oder das (2) Produkt wurde nicht weiter umgesetzt. In diesem Fall wurde zunächst Mesitylen, später NMP als Standard zugesetzt. Es wurde die Variante (1) in Beispielen 3 und 4 und die Variante (2) in Beispielen 1 und 2 durchgeführt.

Im Falle der Veresterung (1) wurden die Gefäße nach 30 Minuten Rühren geöffnet und mit Hilfe von Einwegpipetten wurden 0,25 mL Proben der Reaktionsmischung in 6 mL-Rollrandgefäße transferiert, die 2,0 mL Ethylacetat und 2,0 mL einer wässrigen Natriumhydrogencarbonat-Lösung enthielten. Die beiden Phasen wurden mit Hilfe der Pipette zunächst durchmischt und dann die Phasentrennung abgewartet. Anschließend wurden jeweils 1,0 mL der organischen Phasen über 0,3 mL wasserfreies Magnesiumsulfat in ein 2,0 mL-Probegläschen filtriert und mit jeweils 0,5 mL Lösungsmittel gewaschen. Dabei wurden Einwegpipetten als Filter verwendet, die mit einem Wattepfropfen versehen waren.

Im Falle der Aufarbeitung der freien Säure (2) wurde erst das Lösungsmittel DMSO durch Filtration entfernt. Der Feststoff wurde unter Eisbadkühlung in 5,0 mL destilliertes Wasser aufgenommen und es wurden 50-100 µL N-Methylpyrrolidon (NMP) hinzugefügt. Die klare Lösung wurde zur Bestimmung des Umsatzes in ein 2,0 mL-Probengläschen überführt und mittels HPLC relativ zum internen Standard bestimmt und mit einem Response-Faktor korrigiert.

Die Ergebnisse der Versuche sind in der nachstehenden Tabelle wiedergegeben.

**Versuche mit Acetylen**

| **Beispiel** | **Katalysator** | **Base** | **CO₂-Druck [bar]** | **Ausbeute [%]** | **TON** |
|---|---|---|---|---|---|
| 1 | 0.429 mg [0.0025 mmol] AgNO₃ | 365 mg [2.40 mmol] DBU | 5 | | 5 |
| 2 | 1.07 mg [0.0055 mmol]ppm Cul * | 365 mg [2.40 mmol] DBU | 5 | 7 | 7 |
| Versuche mit 1-Oktin (Vergleich): | | | | | |
| 3 | 1.07 mg [0.0055 mmol] CuI * | 365 mg [2.40 mmol] DBU | 20 | 75 | 300 |
| 4 | 0.0858 mg [0.0005 mmol] AgNO₃ | 365 mg [2.40 mmol] DBU | 20 | 71 | 1418 |

| | | | | | |
|---|---|---|---|---|---|
| * Vergleich | | | | | |

Die Resultate verdeutlichen, dass die Silber-katalysierte direkte Carboxylierung von Acetylen mit CO₂ in Gegenwart von Aminbasen möglich ist.

## Patentansprüche

1. Verfahren zur Herstellung von Acetylendicarbonsäure durch Umsetzung von Acetylen mit Kohlenstoffdioxid, **dadurch gekennzeichnet, dass** die Umsetzung in Gegenwart eines Silberkatalysators und einer Aminbase durchgeführt wird.

2. Verfahren nach Anspruch 1, **dadurch gekennzeichnet, dass** der Silberkatalysator ausgewählt ist aus der Gruppe bestehend aus Silber(I)halogeniden, Silber(I)nitrat und Silbertetrafluoroborat.

3. Verfahren nach einem der Ansprüche 1 bis 2, **dadurch gekennzeichnet, dass** die Aminbase ausgewählt ist aus der Gruppe bestehend aus 1,5-Diazabicyclo[4.3.0]-non-5-en (DBN) und 1,8-Diazabicyclo[5.4.0]-undec-7-en (DBU).

4. Verfahren nach einem der Ansprüche 1 bis 3, **dadurch gekennzeichnet, dass** die Umsetzung in einem Lösungsmittel durchgeführt wird.

5. Verfahren nach einem der Ansprüche 1 bis 4, **dadurch gekennzeichnet, dass** die Umsetzung bei einem Gesamtdruck von 1 - 50 bar und einer Temperatur von 50 bis 120 °C durchgeführt wird.

6. Verfahren nach einem der Ansprüche 1 bis 5, **dadurch gekennzeichnet, dass** die Umsetzung mit einem Molverhältnis Kohlenstoffdioxid zu Acetylen von 2 : 1 bis 50 : 1 durchgeführt wird.

## Claims

1. A method for producing acetylenedicarboxylic acid by reaction of acetylene with carbon dioxide, wherein the reaction is carried out in the presence of a silver catalyst and an amine base.

2. The method according to claim 1 wherein the silver catalyst is selected from the group consisting of silver(I) halides, silver(I) nitrate and silver tetrafluoroborate.

3. The method according to any of claims 1 to 2 wherein the amine base is chosen from the group consisting of 1,5-diazabicyclo[4.3.0]non-5-ene (DBN) and 1,8-diazabicyclo[5.4.0]undec-7-ene (DBU).

4. The method according to any of claims 1 to 3 wherein the reaction is carried out in a solvent.

5. The method according to any of claims 1 to 4 wherein the reaction is carried out at a total pressure of 1-50 bar and a temperature of 50 to 120°C.

6. The method according to any of claims 1 to 5 wherein the reaction is carried out with a molar ratio of carbon dioxide to acetylene of 2:1 to 50:1.

## Revendications

1. Procédé pour la préparation d'acide acétylènedicarboxylique par transformation d'acétylène avec du dioxyde de carbone, **caractérisé en ce que** la transformation est réalisée en présence d'un catalyseur à base d'argent et d'une base de type amine.

2. Procédé selon la revendication 1, **caractérisé en ce que** le catalyseur à base d'argent est choisi dans le groupe constitué par les halogénures d'argent (I), le nitrate d'argent (I) et le tétrafluoroborate d'argent.

3. Procédé selon l'une quelconque des revendications 1 à 2, **caractérisé en ce que** la base de type amine est choisie dans le groupe constitué par le 1,5-diazabicyclo[4.3.0]-non-5-ène (DBN) et le 1,8-diazabicyclo[5.4.0]-undéc-7-ène (DBU).

4. Procédé selon l'une quelconque des revendications 1 à 3, **caractérisé en ce que** la transformation est réalisée dans un solvant.

5. Procédé selon l'une quelconque des revendications 1 à 4, **caractérisé en ce que** la transformation est réalisée à une pression totale de 1-50 bars et à une température de 50 à 120°C.

6. Procédé selon l'une quelconque des revendications 1 à 5, **caractérisé en ce que** la transformation est réalisée à un rapport molaire de dioxyde de carbone à acétylène de 2:1 à 50:1.
